(19) Europäisches Patentamt
European Patent Office
Office européen des brevets

(11) Veröffentlichungsnummer: **0 092 757**
**A1**

(12) # EUROPÄISCHE PATENTANMELDUNG

(21) Anmeldenummer: 83103709.8

(22) Anmeldetag: 16.04.83

(51) Int. Cl.³: C 07 D 231/18
A 01 N 47/18

(30) Priorität: 27.04.82 DE 3215590

(43) Veröffentlichungstag der Anmeldung:
02.11.83 Patentblatt 83/44

(84) Benannte Vertragsstaaten:
CH DE FR GB LI

(71) Anmelder: BAYER AG
Zentralbereich Patente, Marken und Lizenzen
D-5090 Leverkusen 1, Bayerwerk(DE)

(72) Erfinder: Maurer, Fritz, Dr.
Roeberstrasse 8
D-5600 Wuppertal 1(DE)

(72) Erfinder: Hammann, Ingeborg, Dr.
Lutherstrasse 22
D-4330 Mülheim/Ruhr(DE)

(72) Erfinder: Homeyer, Bernhard, Dr.
Obere Strasse 28
D-5090 Leverkusen 3(DE)

(72) Erfinder: Stendel, Wilhelm, Dr.
In den Birken 55
D-5600 Wuppertal 1(DE)

(54) **N-substituierte O-Pyrazol-4-yl-carbaminsäureester, Verfahren zu ihrer Herstellung und ihre Verwendung als Schädlingsbekämpfungsmittel.**

(57) Die vorliegende Erfindung betrifft neue N-substituierte O-pyrazol-4-yl-carbaminsäureester der Formel (I)

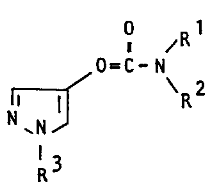

| | |
|---|---|
| $R^1$ und $R^2$ | gleich oder verschieden sind und für Alkyl, Alkenyl, Alkinyl oder Cycloalkyl stehen oder zusammen mit dem Stickstoffatom, an das sie gebunden sind, einen Ring bilden, der weitere Heteroatome enthalten kann, und |
| $R^3$ | für Alkyl, Alkenyl, Alkinyl, Cycloalkyl, Aralkyl oder Aryl steht. |

Weiterhin wurde gefunden, daß man die neuen N-substituierten O-Pyrazol-4-ylcarbaminsäureester der Formel (I) erhält, wenn man 4-Hydroxypyrazole der Formel (II)

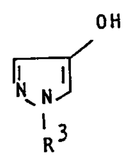

(II)

carbamoyliert.

EP 0 092 757 A1

0092757



BAYER AKTIENGESELLSCHAFT 5090 Leverkusen, Bayerwerk

Zentralbereich
Patente, Marken und Lizenzen

Rt/Büt
Ib

# N-substituierte O-Pyrazol-4-yl-carbaminsäureester, Verfahren zu ihrer Herstellung und ihre Verwendung als Schädlingsbekämpfungsmittel

Die Erfindung betrifft neue N-substituierte O-Pyrazol-4-ylcarbaminsäureester, mehrere Verfahren zu ihrer Herstellung und ihre Verwendung als Schädlingsbekämpfungsmittel, insbesondere als Insektizide.

Es ist bereits bekannt, daß bestimmte Carbaminsäureester, wie z.B. N,N-Dimethyl-O-(1-isopropyl-3-methyl-pyrazol-5-yl)-carbaminsäureester pestizid wirksam sind (vgl.: CH-PS 279 553).

0092757



Die Wirkung dieser Verbindungen ist jedoch, insbesondere bei niedrigen Wirkstoffkonzentrationen und Aufwandmengen, nicht immer ganz zufriedenstellend.

Es wurden neue N-substituierte O-Pyrazol-4-yl-carbaminsäureester der Formel (I)

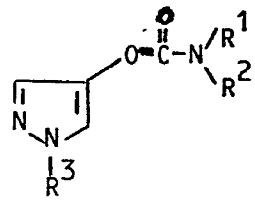

(I)

gefunden, in welcher

$R^1$ und $R^2$ gleich oder verschieden sind und für Alkyl, Alkenyl, Alkinyl oder Cycloalkyl stehen oder zusammen mit dem Stickstoffatom, an das sie gebunden sind, einen Ring bilden, der weitere Heteroatome enthalten kann, und

$R^3$ für Alkyl, Alkenyl, Alkinyl, Cycloalkyl, Aralkyl oder Aryl steht.

Weiterhin wurde gefunden, daß man die neuen N-substituierten O-Pyrazol-4-ylcarbaminsäureester der Formel (I) erhält, wenn man 4-Hydroxypyrazole der Formel (II)

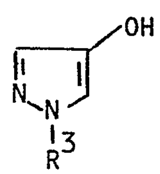

(II)

in welcher

$R^3$ die oben angegebene Bedeutung hat,

a) gegebenenfalls in Gegenwart eines Verdünnungsmittels und gegebenenfalls in Gegenwart eines Säurebinders mit Carbamoylhalogeniden der Formel (III)

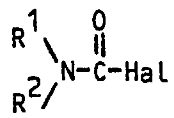

(III),

in welcher

$R^1$ und $R^2$ die oben angegebene Bedeutung haben und

Hal für Fluor oder Chlor steht,

umsetzt, oder

b) gegebenenfalls in Gegenwart eines Verdünnungsmittels und gegebenenfalls in Gegenwart eines Säurebinders zunächst mit Phosgen und danach mit Aminen der Formel (IV)

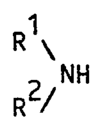

(IV)

in welcher $R^1$ und $R^2$ die oben angegebene Bedeutung haben, umsetzt.

Die neuen N-substituierten O-Pyrazol-4-ylcarbaminsäureester der Formel (I) verfügen über Eigenschaften, die ihre Verwendung als Schädlingsbekämpfungsmittel ermöglichen, insbesondere zeichnen sie sich durch eine hervorragende insektizide Wirksamkeit aus.

Ueberraschenderweise zeigen die erfindungsgemäßen neuen N-substituierten O-Pyrazol-4-yl-carbaminsäureestereine wesentlich bessere insektizide Wirkung als die nach dem Stand der Technik bekannten Verbindungen analoger Konstitution und gleicher Wirkungsrichtung wie z.B. N,N-Dimethyl-O-(1-isopropyl-3-methyl-pyrazol-5-yl)-carbaminsäureester.

Die erfindungsgemäßen N-substituierten O-Pyrazol-4-yl-carbaminsäureester sind durch die Formel (I) allgemein definiert. In dieser Formel stehen vorzugsweise

$R^1$ und $R^2$, welche gleich oder verschieden sein können, für geradkettiges oder verzweigtes Alkyl mit 1 bis 5 Kohlenstoffatomen, Alkenyl oder Alkinyl mit jeweils 2 bis 5 Kohlenstoffatomen; für Cycloalkyl mit 3 bis 7 Kohlenstoffatomen oder zusammen mit dem Stickstoffatom, an das sie gebunden sind, für einen 5- oder 6-gliedrigen Ring, der N oder O als weiteres Heteroatom enthalten kann, sowie

$R^3$ für geradkettiges oder verzweigtes Alkyl, mit 1 bis 6 Kohlenstoffatomen, Alkenyl oder Alkinyl mit jeweils 2 bis 6 Kohlenstoffatomen; für Cycloalkyl mit 3 bis 7 Kohlenstoffatomen; für Aryl mit 6 bis 10 Kohlenstoffatomen oder für Aralkyl mit 1 bis 2 Kohlenstoffatomen im Alkylteil und 6 bis 10 Kohlenstoffatomen im Arylteil.

Besonders bevorzugt sind diejenigen N-substituierten O-Pyrazol-4-yl-carbaminsäureester der Formel (I), in denen

$R^1$ und $R^2$, welche gleich oder verschieden sein können, für Methyl, Ethyl, n- und iso-Propyl, n-, iso-, sec- und t-Butyl, 2-Methylbutyl, Alkyl, Propargyl, Cyclopropyl, Cyclobutyl, Cyclopentyl und Cyclohexyl stehen, oder zusammen mit dem Stickstoffatom, an das sie gebunden sind, für 1-Pyrrolidinyl, 4-Methyl-1-piperazinyl und 4-Morpholinyl stehen, und

$R^3$ für Methyl, Ethyl, n- und iso-Propyl, n-, iso-, sec- und t-Butyl, 1,1-Dimethylpropyl, Alkyl, Propargyl, Cyclopropyl, Cyclobutyl, Cyclopentyl, Cyclohexyl, Benzyl und Phenyl steht.

Besonders bevorzugte Einzelverbindungen sind die bei den Herstellungsbeispielen genannten Verbindungen.

Verwendet man als Ausgangsstoffe beispielsweise 1-Isopropyl-4-hydroxypyrazol und N,N-Dimethyl-carbamoylchlorid, so kann der Reaktionsablauf durch das folgende Formelschema wiedergegeben werden :

(Verfahren a)

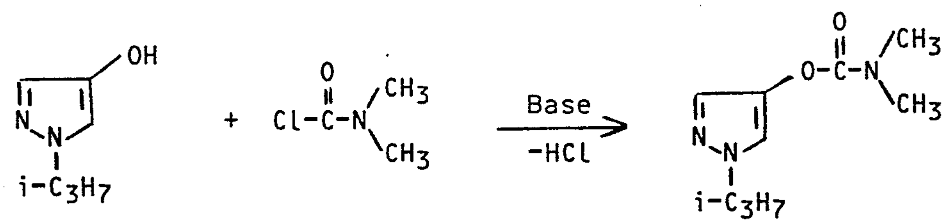


Verwendet man als Ausgangsstoffe beispielsweise 1-(t-Butyl)-4-hydroxypyrazol und Phosgen sowie Methyl-ethyl-amin, so kann der Reaktionsablauf durch das folgende Formelschema wiedergegeben werden :

(Verfahren b)

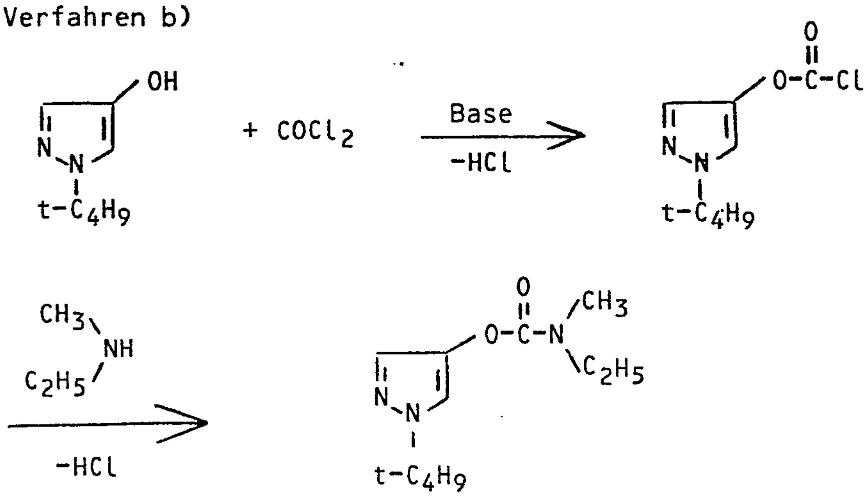

Die für die erfindungsgemäßen Verfahren (a) und (b) als Ausgangsstoffe zu verwendenden 4-Hydroxypyrazole sind durch die Formel (II) allgemein definiert. In dieser Formel steht $R^3$ vorzugsweise für diejenigen Reste, die bereits im Zusammenhang mit der Beschreibung der erfindungsgemäßen Stoffe der Formel (I) vorzugsweise für diesen Substituenten genannt werden. Als Beispiele seien genannt : 1-Methyl-, 1-Ethyl-, 1-n-Propyl , 1-Isopropyl-, 1-n-Butyl-, 1-Isobutyl-, 1-(2-Butyl)-, 1-t-Butyl-, 1-(1,1-Dimethylpropyl)-, 1-Alkyl, 1-Propargyl, 1-Cyclohexyl-, 1-Cyclopentyl-, 1-Cyclobutyl-, 1-Cyclopropyl-, 1-Benzyl- und 1-Phenyl-4-hydroxypyrazol.

Die 4-Hydroxy-pyrazole der Formel (II) sind teilweise bekannt (vgl. Liebigs Ann.Chem. 313, (1900), 17). Man erhält sie beispielsweise durch Umsetzung von bekannten 4-Methoxy-pyrazolen mit Bromwasserstoffsäure. Die Herstellung der 4-Methoxy-pyrazole erfolgt auf bekannte Weise aus Hydrazin und 2-Methoxy-4-dimethylamino-acrolein (vgl. Archiv der Pharmazie 300 (1967), 704-708).

Die für das erfindungsgemäße Verfahren (a) weiterhin als Ausgangsstoffe zu verwendenden Carbamoylhalogenide sind durch die Formel (III) allgemein definiert. In dieser Formel stehen $R^1$ und $R^2$ vorzugsweise für die Reste, die bereits im Zusammenhang mit der Beschreibung der erfindungsgemäßen Stoffe der Formel (I) vorzugsweise für diese Substituenten genannt wurden. Hal steht vorzugsweise für Fluor oder Chlor.

Als Beispiele seien genannt : N,N-Dimethyl-, N,N-Diethyl-, N-Methyl-N-ethyl-, N-Methyl-N-isopropyl-, N,N-Diisopropyl-, N,N-Di-n-butyl-, N,N-Diisobutyl-, N,N-Diisopentyl- und N,N-Tetramethylen-carbamoylfluorid bzw. -chlorid.

Die Carbamoylhalogenide der Formel (III) sind bekannt oder lassen sich nach allgemein üblichen und bekannten Verfahren herstellen.

Die für das erfindungsgemäße Verfahren (b) weiterhin als Ausgangsstoffe zu verwendenden Amine sind durch die Formel (IV) allgemein definiert. In dieser Formel stehen $R^1$ und $R^2$ vorzugsweise für die Reste, die bereits im Zusammenhang mit der Beschreibung der erfindungsgemäßen Stoffe der Formel (I) vorzugsweise für diese Substituenten genannt wurden.

Als Beispiel seien genannt : N,N-Dimethyl-, N,N-Diethyl-, N-Methyl-N-ethyl-, N-Methyl-N-isopropyl-, N-Methyl-N-allyl-, N-Methyl-N-propargyl-, N,N-Diisopropyl-, N,N-Di-n-butyl-, N,N-Diisobutyl-, N,N-Diisopentyl-amin, Pyrrolidin, Morpholin und Piperazin.

Die Amine der Formel (IV) sind allgemein bekannte Verbindungen der organischen Chemie.

Als Verdünnungsmittel kommen für die Umsetzung gemäß Verfahrensvariante (a) vorzugsweise alle inerten organischen Lösungsmittel infrage. Hierzu gehören insbesondere aliphatische und aromatische, gegebenenfalls chlorierte Kohlenwasserstoffe, wie Benzin, Benzol, Toluol, Xylol, Methylenchorid, Chloroform,. Tetrachlorkohlenstoff, Chlorbenzol und o-Dichlorbenzol, Ether wie Diethylether und Dibutylether, Tetrahydrofuran und Dioxan, Ketone wie Aceton, Methylethyl- und Methylisopropyl- und Methylisobutylketon sowie Nitrile, wie Acetonitril und Propionitril.

Die Umsetzung nach Verfahren (a) wird in Gegenwart von Säurebindern vorgenommen. Man kann alle üblicherweise verwendbaren anorganischen und organischen Säurebinder zugeben. Hierzu gehören vorzugsweise Alkalicarbonate,wie beispielsweise Natriumcarbonat, Kaliumcarbonat und Natriumhydrogencarbonat,ferner niedere tertiäre Alkylamine, Cycloalkylamine oder Arylalkylamine, wie beispielsweise Triethylamin, N,N-Dimethyl-benzylamin, weiterhin Pyridin sowie 1,4-Diazabicyclo(2,2,2)-octan und1,5-Diazabicyclo(4,3,0)-non-5-en.

Die Reaktionstemperatur kann bei Verfahren (a) innerhalb eines größeren Bereiches variiert werden. Im allgemeinen arbeitet man zwischen 0 und 100°C, vorzugsweise zwischen 10 und 80°C.

Das Verfahren (a) wird im allgemeinen bei Normaldruck durchgeführt. Zur Durchführung des erfindungsgemäßen Verfahrens (a) setzt man auf 1 Mol 4-Hydroxy-pyrazol der Formel (II) gewöhnlich zwischen 1 und 1,5 Mol, vorzugsweise zwischen 1 und 1,2 Mol Carbamoylhalogenid der Formel (III) ein.

Die Umsetzung wird vorzugsweise unter Verwendung eines der oben genannten Säurebindern in einem der oben angegebenen Verdünnungsmittel durchgeführt. Das Reaktionsgemisch wird mehrere Stunden bei der erforderlichen Temperatur gerührt. Dann wird das Lösungsmittel im Vakuum abdestilliert. Man erhält so die Produkte in öliger oder kristalliner Form. Zur Charakterisierung dient der Schmelzpunkt oder der Brechungsindex.

Für die Durchführung gemäß Verfahrensvariante (b) kommen als Verdünnnungsmittel vorzugsweise alle inerten organischen Lösungsmittel infrage. Hierzu gehören vorzugsweise die bei Verfahren (a) aufgezählten Solventien.

Die Umsetzung nach Verfahren (b) wird in Gegenwart von Säurebindern vorgenommen. Man kann alle üblicherweise verwendbaren anorganischen und organischen Säurebinder zugeben. Hierzu gehören vorzugsweise Alkalicarbonate, wie beispielsweise Natriumcarbonat, Kaliumcarbonat und Natriumhydrogencarbonat, ferner niedere tertiäre Alkylamine, Cycloalkylamine oder Arylalkylamine, wie beispielsweise Triethylamin, N,N-Dimethyl-benzylamin, weiterhin Pyridin sowie 1,4-Diazabicyclo(2,2,2)-octan und 1,5-Diazabicyclo(4,3,0)-non-5-en.

Die Reaktionstemperaturen können bei Verfahren (b) innerhalb eines größeren Bereiches variiert werden. Im allgemeinen arbeitet man zwischen -20°C und +80°C, vorzugsweise zwischen 0°C und 60°C.

Das Verfahren (b) wird im allgemeinen bei Normaldruck durchgeführt. Zur Durchführung des erfindungsgemäßen Verfahrens (b) setzt man auf 1 Mol 4-Hydroxypyrazol der Formel (II) gewöhnlich zwischen 1 und 1,5 Mol, vorzugsweise zwischen 1 und 1,2 Mol Phosgen sowie zwischen 1 und 1,5 Mol, vorzugsweise zwischen 1 und 1,2 Mol Amin der Formel (IV) ein.

Die Umsetzung wird vorzugsweise unter Verwendung von einem der oben genannten Säurebinder in einem der oben angegebenen Verdünnungsmittel durchgeführt.

Das Reaktionsgemisch wird mehrere Stunden bei der erforderlichen Temperatur gerührt. Die Aufarbeitung erfolgt in der üblichen Art und Weise. Zur Charakterisierung dient der Schmelzpunkt oder der Brechungsindex.

Die erfindungsgemäßen N-substituierten O-Pyrazol-4-yl-carbaminsäureester zeichnen sich durch hervorragende insektizide Wirksamkeit aus.

Die Wirkstoffe eignen sich bei guter Pflanzenverträglichkeit und günstiger Warmblütertoxizität zur Bekämpfung von tierischen Schädlingen, insbesondere Insekten, ganz besonders bevorzugt zur Bekämpfung von Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia. Aus der Ordnung der Isoptera z.B. Reticulitermes spp.. Aus der Ordnung der Anoplura z.B. Phylloxera vastatrix, Pemphigus spp., Pediculus humanus corporis, Haematopinus spp., Linognathus spp. Aus der Ordnung der Mallophaga z.B. Trichodectes spp., Damalinea spp. Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp. Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp. Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris

spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attegenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordung der Hymenoptera z.B. Diprion spp., Hoplocampa spp., Lasius spp., Monomorium pharaonis, Vespa spp..

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinelle frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die neuen Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo - und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird,

ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Wie bereits oben angegeben, sind die neuen erfindungsgemäßen Wirkstoffe erhältlichen bekannten Wirkstoffen überlegen.

Herstellungsbeispiele :

Beispiel 1 :

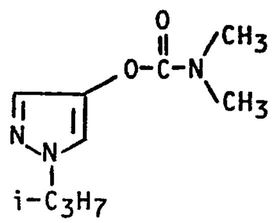

(Verfahren a)

Eine Mischung aus 8,8 g (0,07 Mol) 1-Isopropyl-4-hydroxypyrazol (DE-OS 2 931 033), 13,8 g (0,07 Mol) Kaliumcarbonat, 150 ml Acetonitril und 8 g (0,07 Mol) N,N-Dimethylcarbamidsäurechlorid wird über 4 Stunden bei 60°C gerührt. Dann kühlt man auf 20°C bis 25°C ab, filtriert vom anorganischen Material und dampft das Filtrat im Vakuum ein. Der Rückstand wird im Hochvakuum andestilliert. Man erhält 13 g (95 % der Theorie) N,N-Dimethyl-O-(1-isopropyl-pyrazol-4-yl)-carbamidsäureester vom Brechungsindex $n_D^{21}$ : 1,4868.

Beispiel 2 :

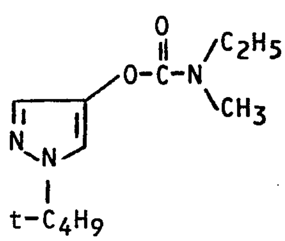

(Verfahren b)

Zu einer Lösung von 11 g (0,11 Mol) Phosgen in 150 ml Toluol tropft man bei 0°C bis 5°C zuerst 10,1 g (0,1 Mol) Triethylamin und dann eine Lösung von 14 g (0,1 Mol) 1-t-Butyl-4-hydroxypyrazol (DE-OS 2 931 033) in 150 ml Toluol. Man rührt das Reaktionsgemisch eine

Stunde bei 0°C bis 5°C nach, dann läßt man die Temperatur bis ca. 20°C steigen und gibt ein Gemisch aus 5,9 g (0,1 Mol) Methyläthylamin und 10,1 g (0,1 Mol) Triäthylamin zu.

Man rührt das Gemisch 20 Stunden ohne Kühlung, wäscht es dann zweimal mit jeweils 50 ml Wasser und trocknet die organische Phase über Natriumsulfat. Das Lösungsmittel wird im Vakuum abdestilliert und der Rückstand im Hochvakuum andestilliert. Man erhätl ca. 21 g (93 % der Theorie) N-Methyl-N-ethyl-O-(1-t-butylpyrazol-4-yl)-carbamidsäureester vom Brechungsindex $n_D^{24}$ : 1,4818.

Entsprechend Beispiel 1 und Beispiel 2 sowie entsprechend den Verfahrensvarianten (a) und (b) werden die Verbindungen der allgemeinen Formel

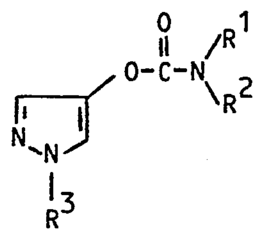

(I)

erhalten.

| Beisp. Nr. | $R^1$ | $R^2$ | $R^3$ | Schmelzpunkt (°C) oder Brechungsindex |
|---|---|---|---|---|
| 3 | $CH_3$ | $CH_3$ | $C_2H_5$ | $n_D^{21}$ 1,4920 |
| 4 | $CH_3$ | $CH_3$ | sec-$C_4H_9$ | $n_D^{21}$ 1,4851 |
| 5 | $CH_3$ | $C_2H_5$ | i-$C_3H_7$ | $n_D^{24}$ 1,4839 |
| 6 | $CH_3$ | i-$C_3H_7$ | i-$C_3H_7$ | $n_D^{24}$ 1,4822 |
| 7 | $CH_3$ | i-$C_3H_7$ | t-$C_4H_9$ | $n_D^{24}$ 1,4802 |
| 8 | $CH_3$ | $CH_3$ | t-$C_4H_9$ | $n_D^{20}$ 1,4845 |
| 9 | $CH_3$ | $CH_3$ | $CH_3-CH_2-C(CH_3)_2-$ | $n_D^{20}$ 1,4831 |
| 10 | $C_2H_5$ | $C_2H_5$ | i-$C_3H_7$ | $n_D^{19}$ 1,4809 |
| 11 | $C_2H_5$ | $C_2H_5$ | t-$C_4H_9$ | 54 |
| 12 | i-$C_3H_7$ | i-$C_3H_7$ | i-$C_3H_7$ | $n_d^{19}$ 1,4868 |
| 13 | $-CH_2-CH_2-CH_2-CH_2-$ | | i-$C_3H_7$ | $n_D^{19}$ 1,5069 |
| 14 | $-CH_2-CH_2-O-CH_2-CH_2-$ | | i-$C_3H_7$ | $n_D^{19}$ 1,5061 |
| 15 | $-CH_2-CH_2-O-CH_2-CH_2-$ | | t-$C_4H_9$ | $n_D^{19}$ 1,5045 |
| 16 | $-CH_2-CH_2-CH_2-CH_2-$ | | t-$C_4H_9$ | $n_D^{19}$ 1,5034 |

Tabelle (Fortsetzung)

| Beisp. Nr. | $R^1$ | $R^2$ | $R^3$ | Schmelzpunkt (°C) oder Brechungsindex |
|---|---|---|---|---|
| 17 | $n-C_3H_7$ | $n-C_3H_7$ | $i-C_3H_7$ | $n_D^{19}$ 1,4785 |
| 18 | $-CH_2-CH_2-N(CH_3)-CH_2-CH_2-$ | | $i-C_3H_7$ | $n_D^{19}$ 1,5079 |
| 19 | $CH_3$ | 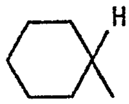 | $i-C_3H_7$ | $n_D^{19}$ 1,5018 |
| 20 | $n-C_3H_7$ | $n-C_3H_7$ | $t-C_4H_9$ | $n_D^{20}$ 1,4785 |
| 21 | $-CH_2-CH_2-N(CH_3)-CH_2-CH_2-$ | | $t-C_4H_9$ | 97 |
| 22 | $CH_3$ | 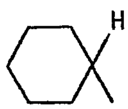 | $t-C_4H_9$ | $n_D^{19}$ 1,5009 |
| 23 | $C_2H_5$ | $i-C_3H_7$ | $i-C_3H_7$ | $n_D^{19}$ 1,4852 |
| 24 | $C_2H_5$ | $i-C_3H_7$ | $t-C_4H_9$ | $n_D^{19}$ 1,4832 |
| 25 | $CH_3$ | $-CH_2-CH=CH_2$ | $i-C_3H_7$ | $n_D^{19}$ 1,4921 |
| 26 | $CH_3$ | $-CH_2-CH=CH_2$ | $t-C_4H_9$ | $n_D^{19}$ 1,4913 |
| 27 | $CH_3$ | $t-C_4H_9$ | $i-C_3H_7$ | $n_D^{19}$ 1,4886 |
| 28 | $CH_3$ | $t-C_4H_9$ | $t-C_4H_9$ | $n_D^{19}$ 1,4889 |

## Herstellung der Ausgangsprodukte

### Ausgangsprodukt 1

Die als Ausgangsmaterialien zu verwendenden 4-Hydroxy-pyrazole können z.B. wie folgt hergestellt werden:

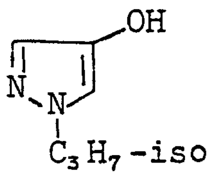

Eine Lösung von 27,6 g (0,2 Mol) 1-iso-Propyl-4-methoxy-pyrazol (Darstellung s.Archiv der Pharmazie 300(1967), S.704-708) in 100 ml 48%-iger Bromwasserstoffsäure wird 9 Stunden unter Rückfluß gekocht. Dann destilliert man die überschüssige Säure im Vakuum ab, löst den Rückstand in 40 ml Wasser und neutralisiert die Lösung durch Zugabe von festem Natriumhydrogencarbanat. Anschließend extrahiert man 6 x mit je 40 ml Chloroform, trocknet die vereinigten Auszüge über Natriumsulfat und destilliert das Lösungsmittel im Vakuum ab. Zurück bleiben 18,6g (75 % der Theorie) 1-iso-Propyl-4-hydroxy-pyrazol in Form beiger Kristalle mit dem Schmelzpunkt 63°C.

In analoger Weise werden die übrigen 4-Hydroxy-pyrazole der Formel (II) hergestellt.

Ausgangsprodukt 2

Die teilweise bekannten 4-Methoxypyrazole, die als Vorprodukte für die 4-Hydroxy-pyrazole eingesetzt werden, können z,B. wie folgt hergestellt werden:

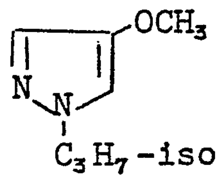

Eine Gemisch von 129g (1 Mol) 2-Methoxy-3-dimethylaminoacrolein (Darstellung s.Archiv der Pharmazie 300 (1967), S. 704-708), 123 g (1 Mol) Isopropylhydrazinhemisulfat und 1,1 Mol einer Lösung von Natriummethylat in 400 ml Methanol wird 24 Stunden unter Rückfluß gekocht. Dann destilliert man das Lösungsmittel im Vakuum ab, versetzt den Rückstand mit 500 ml Wasser und extrahiert dann mit 1 l Chloroform. Die organische Lösung wird mit 500 ml Wasser gewaschen, über Natriumsulfat getrocknet und dann bei 30°C im Vakuum eingedampft. Den Rückstand destilliert man im Vakuum und erhält so 82,5g (60 % der Theorie) 1-Isopropyl-4-methoxypyrazol in Form eines gelben Oels mit dem Siedepunkt 94-96°C/ 14 Torr.

In analoger Weise werden die übrigen 4-Methoxy-pyrazole hergestellt.

Anwendungsbeispiele :

In den nachfolgenden Beispielen wird folgende Verbindung als Vergleichssubstanz eingesetzt :

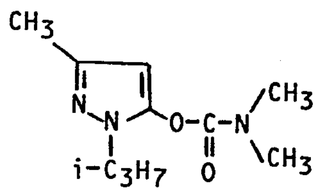

(A)

N,N-Dimethyl-O-(1-isopropyl-3-methyl-pyrazol-5-yl)-carbaminsäureester.

## Beispiel A

Laphygma-Test

Lösungsmittel: 3 Gewichtsteile Dimethylformamid

Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentrationen behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2, 3, 4, 5, 6, 7, 8, 9.

Beispiel B

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2, 5, 7, 8, 9, 10, 11.

Beispiel C

Grenzkonzentrations-Test / Wurzelsystemische Wirkung

| | |
|---|---|
| Testinsekt: | Myzus persicae |
| Lösungsmittel: | 3 Gewichtsteile Aceton |
| Emulgator: | 1 Gewichtsteil Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigt z.B. die folgende Verbindung der Herstellungsbeispiele überlegene Wirkung gegenüber dem Stand der Technik: 7.

**Beispiel D**

Test mit Boophilus microplus resistent

Lösungsmittel: 35 Gewichtsteile Ethylenglykolmonomethylether
35 Gewichtsteile Nonylphenolpolylglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

lo adulte Boophilus microplus res. werden in die zu testende Wirkstoffzubereitung 1 Minute getaucht. Nach Ueberführung in Plastikbecher und Aufbewahrung in einem klimatisierten Raum wird der Abtötungsgrad bestimmt.

Bei diesem Test zeigt z.B. die folgende Verbindung der Herstellungsbeispiele überlegene Wirkung gegenüber dem Stand der Technik:
1 .

Beispiel E

Test mit Lucilia cuprina res.-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 2o Lucilia cuprina res.-Larven werden in ein Teströhrchen gebracht, welches ca. 1 $cm^2$ Pferdefleisch und o,5 ml der Wirkstoffzubereitung enthält. Nach 24 Stunden wird der Abtötungsgrad bestimmt.

Bei diesem Test zeigt z.B. die folgende Verbindung der Herstellungsbeispiele überlegene Wirkung gegenüber dem Stand der Technik: 1 .

## Patentansprüche

1. N-substituierte O-Pyrazol-4-yl-carbaminsäureester der Formel I

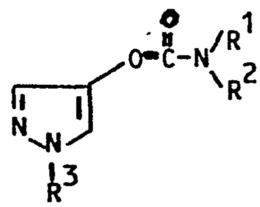

(I)

in welcher

$R^1$ und $R^2$ gleich oder verschieden sind und für Alkyl, Alkenyl, Alkinyl oder Cycloalkyl stehen oder zusammen mit dem Stickstoffatom, an das sie gebunden sind, einen Ring bilden, der weitere Heteroatome enthalten kann, und

$R^3$ für Alkyl, Alkenyl, Alkinyl, Cycloalkyl, Aralkyl oder Aryl steht.

2. Verfahren zur Herstellung der N-substituierten O-Pyrazol-4-yl-carbaminsäureester der Formel (I)

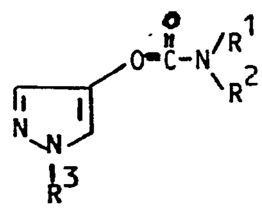

(I)

in welcher

$R^1$ und $R^2$ gleich oder verschieden sind und für Alkyl, Alkenyl, Alkinyl oder Cycloalkyl stehen oder zusammen mit dem Stickstoffatom, an das sie gebunden sind, einen Ring bilden, der weitere Heteroatome enthalten kann, und

$R^3$ für Alkyl, Alkenyl, Alkinyl, Cycloalkyl, Aralkyl oder Aryl steht, dadurch gekennzeichnet, daß man

4-Hydroxypyrazole der Formel (II)

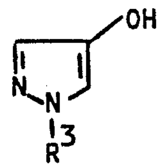

(II)

in welcher

$R^3$ die oben angegebene Bedeutung hat,

a) gegebenenfalls in Gegenwart eines Verdünnungsmittels und gegebenenfalls in Gegenwart eines Säurebinders mit Carbamoylhalogeniden der Formel (III)

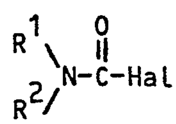

(III),

in welcher

$R^1$ und $R^2$ die oben angegebene Bedeutung haben und

Hal für Fluor oder Chlor steht,

umsetzt, oder

b) gegebenenfalls in Gegenwart eines Verdünnungsmittels und gegebenenfalls in Gegenwart eines Säurebinders zunächst mit Phosgen und danach mit Aminen der Formel (IV),

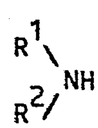

(IV)

in welcher $R^1$ und $R^2$ die oben angegebene Bedeutung haben, umsetzt.

3. N-substituierte O-Pyrazol-4-yl-carbaminsäureester der Formel (I) in welcher

$R^1$ und $R^2$, welche gleich oder verschieden sein können, für geradkettiges oder verzweigtes Alkyl mit 1 bis 5 Kohlenstoffatomen, Alkenyl oder Alkinyl mit jeweils 2 bis 5 Kohlenstoffatomen; für Cycloalkyl mit 3 bis 7 Kohlenstoffatomen oder zusammen mit dem Stickstoffatom, an das sie gebunden sind, für einen 5- oder 6-gliedrigen Ring, der N oder O als weiteres Heteroatom enthalten kann, sowie

$R^3$ für geradkettiges oder verzweigtes Alkyl, mit 1 bis 6 Kohlenstoffatomen, Alkenyl oder Alkinyl mit jeweils 2 bis 6 Kohlenstoffatomen; für Cycloalkyl mit 3 bis 7 Kohlenstoffatomen; für Aryl mit 6 bis 10 Kohlenstoffatomen oder für Aralkyl mit 1 bis 2 Kohlenstoffatomen im Alkylteil und 6 bis 10 Kohlenstoffatomen im Arylteil stehen.

4. N-substituierte O-Pyrazol-4-yl-carbaminsäureester der Formel (I), in welcher

$R^1$ und $R^2$, welche gleich oder verschieden sein können, für Methyl, Ethyl, n- und iso-Propyl, n-, iso-, sec- und t-Butyl, 2-Methylbutyl, Alkyl, Propargyl, Cyclopropyl,Cyclobutyl, Cyclopentyl und Cyclohexyl stehen, oder zusammen mit dem Stickstoffatom, an das sie gebunden sind, für 1-Pyrrolidinyl, 4-Methyl-1-piperazinyl und 4-Morpholinyl stehen, und

$R^3$ für Methyl, Ethyl, n- und iso-Propyl, n-, iso-, sec- und t-Butyl, 1,1-Dimethylpropyl, Alkyl, Propargyl, Cyclopropyl, Cyclobutyl, Cyclopentyl, Cyclohexyl, Benzyl und Phenyl steht.

5. Schädlingsbekämpfungsmittel, gekennzeichnet durch einen Gehalt an mindestens einem N-substituierten O-Pyrazolyl-4-yl-carbaminsäureester der Formel (I).

6. Verwendung von N-substituiertem O-Pyrazolyl-4-yl-carbaminsäureester der Formel (I) zur Bekämpfung von Schädlingen.

7. Verfahren zur Bekämpfung von Schädlingen, dadurch gekennzeichnet, daß man N-substituierte O-Pyrazolyl-4-yl-carbaminsäureester der Formel (I) auf Schädlinge und/oder ihren Lebensraum einwirken läßt.

8. Verfahren zur Herstellung von Schädlingsbekämpfungsmitteln, dadurch gekennzeichnet, daß man N-substituierte O-Pyrazolyl-4-yl-carbaminsäureester der Formel (I) mit Streckmitteln und/oder oberflächenaktiven Mitteln vermischt.

0092757

Europäisches Patentamt

# EUROPÄISCHER RECHERCHENBERICHT

Nummer der Anmeldung

EP 83 10 3709

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| P,X | CENTRAL PATENTS INDEX - BASIC ABSTRACTS JOURNAL, Section C: AGDOC, Woche E30, 22. September 1982, Referenz Nr. 62304, Derwent Publications Ltd., Rochdale House, London, GB. & JP - A - 57 098 267 (TAKEDA CHEMICAL IND. K.K.) 09.12.1980 * Zusammenfassung * --- | 1-8 | C 07 D 231/18<br>A 01 N 47/18 |
| A | EP-A-0 023 326 (BAYER) * Ansprüche * --- | 1-8 | |
| P,A | EP-A-0 062 821 (BAYER) * Ansprüche * ----- | 1-8 | |
| | | | RECHERCHIERTE SACHGEBIETE (Int. Cl. 3) |
| | | | C 07 D 231/00<br>A 01 N 47/00 |

Der vorliegende Recherchenbericht wurde für alle Patentansprüche erstellt.

| Recherchenort | Abschlußdatum der Recherche | Prüfer |
|---|---|---|
| DEN HAAG | 23-06-1983 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82